# FASCICULE DE BREVET EUROPEEN

(11) **EP 3 047 259 B1**
(45) Date de publication et mention de la délivrance du brevet: **11.08.2021**
(21) Numéro de dépôt: 14777725.4
(22) Date de dépôt: 08.09.2014
(51) Int. Cl.: G01N 21/85, G01N 33/18

(54) **PROCEDE ET DISPOSITIF D'ANALYSE DE SULFATES DANS UN LIQUIDE**
VERFAHREN UND VORRICHTUNG ZUR ANALYSE VON SULFATEN IN EINER FLÜSSIGKEIT
METHOD AND DEVICE FOR ANALYSING SULFATES IN A LIQUID

(30) Priorité: 16.09.2013 FR 1358912
(43) Date de publication de la demande: 27.07.2016
(73) Titulaire: Institut Francais de Recherche pour l'Exploitation de la Mer (IFREMER), 92130 Issy-les-Moulineaux (FR)
(72) Inventeur: CARADEC, Florian, F-29190 Lennon (FR); LAËS-HUON, Agathe, F-29200 Brest (FR); CHATAING-PARIAUD, Sophie, F-29280 Locmaria-Plouzane (FR); BUCAS, Kareen, F-29870 Lannilis (FR)
(74) Mandataire: Novagraaf Technologies
(86) Numéro de dépôt international: PCT/FR2014/052214
(87) Numéro de publication internationale: WO 2015/036680

(56) Documents cités:
- GB-A- 1 175 967
- VUILLEMIN R ET AL: "CHEMINI: A new in situ CHEmical MINIaturized analyzer", DEEP SEA RESEARCH. PART 1. OCEANOGRAPHIC RESEARCH PAPERS, PERGAMON PRESS, OXFORD, GB, vol. 56, no. 8, 1 août 2009 (2009-08-01), pages 1391-1399, XP026221521, ISSN: 0967-0637, DOI: 10.1016/J.DSR.2009.02.002 [extrait le 2009-02-07] cité dans la demande
- None

## Description

### Domaine technique

La présente invention se rapporte à un dispositif d'analyse de la quantité de sulfates contenue dans un liquide, ainsi qu'à l'utilisation du dispositif pour l'analyse d'un liquide, notamment d'eau de mer, notamment nanofiltrée.

La présente invention trouve notamment des applications dans les domaines de l'industrie pétrolière offshore, notamment l'exploitation de gisement, par exemple pétroliers, ainsi que dans les procédés industriels tels que la fabrication de papier et de cellulose, la fabrication d'huiles comestibles, les tanneries, la surveillance environnementale, le traitement des eaux, etc.

Dans la description ci-dessous, les références entre crochets (**[ ]**) renvoient à la liste des références présentée à la fin des exemples.

### Etat de la technique

L'industrie pétrolière met en œuvre différentes techniques pour exploiter les gisements dont celle d'injecter de l'eau dans les réservoirs naturels de ces gisements afin d'augmenter la pression et ainsi de favoriser l'extraction du pétrole. L'eau injectée provient généralement de l'eau de mer environnante dans le cas des exploitations offshore.

Cette eau de mer, riche en sulfates, peut entrainer la formation de précipités de sulfates de baryum, de sulfates de strontium et de sulfates de calcium, ces ions étant présents dans les eaux des réservoirs naturels. Ce phénomène engendre de nombreux problèmes sur les installations d'extraction de ces gisements, notamment une baisse de l'efficacité de production ainsi qu'une augmentation des opérations de maintenance de ces installations.

Afin de prévenir ce type de problèmes, l'eau de mer est préalablement désulfatée par un procédé de nanofiltration.

Le taux de sulfates en sortie du procédé de nanofiltration est généralement surveillé pour vérifier la concentration en sulfates des eaux injectées, appelées aussi eaux d'injection. L'opérateur ou le responsable de la maintenance détermine une valeur seuil, par exemple une concentration en sulfates autour de 40 mg.L⁻¹ (0,42x10⁻³ mol/L ou 0,42 mM), déterminant l'arrêt de l'injection des eaux nanofiltrées et le déclenchement d'interventions de maintenance des installations. Ces interventions comprennent par exemple le rinçage des installations, toute partie du dispositif sensible au colmatage, etc.

Elles nécessitent l'arrêt de l'extraction et des moyens humains, de matériel et de réactifs importants. Il est donc essentiel de disposer d'un moyen efficace et économique d'analyse de ces sulfates qui permet d'optimiser ces interventions et d'en minimiser les coûts.

Dans le cas d'une application dans l'industrie pétrolière offshore, de nombreuses contraintes sont à considérer. Dans un premier temps, il est nécessaire de disposer d'une mesure des sulfates en ligne. En effet, un prélèvement manuel puis une analyse n'est pas souhaitable dans ce cas, car ce prélèvement engendre des opérations de maintenance et des coûts supplémentaires, impliquant un besoin en personnel et une contamination possible du liquide à analyser.

En outre, afin de limiter les opérations de maintenance liées au dispositif d'analyse lui-même, il est nécessaire de disposer d'un dispositif d'analyse économique en termes de réactifs et qui présente une autonomie énergétique suffisante pour ne pas être soumis lui-même à des opérations de maintenances chronophages et couteuses.

De plus, il n'existe pas actuellement de dispositifs automatisés pratiques, fiables, adaptés et de taille appropriée pour pouvoir être disposé facilement au sein d'installations d'extraction de gisement.

Les analyseurs de sulfates de l'art antérieur ne remplissent malheureusement pas l'ensemble de ces conditions, car ces analyseurs de l'art antérieur ne sont pas qualifiés pour la mesure dans une matrice en eau de mer. Pour certains, un traitement préalable par résine échangeuse de cations est même nécessaire afin d'éviter les interférences avec d'autres ions tels que les ions calcium ou bien la formation de précipités.

Par ailleurs, les précisions de mesure, ou gammes de mesure, des appareils de l'art antérieur sont relativement limitées. Certaines présentent des limites basses supérieures à 20 mg.L⁻¹ (0,21x10⁻³ mol/L ou 0,21 mM) ou ne sont pas précises pour les faibles valeurs en sulfates, par exemple de 0 à 20 mg.L⁻¹ (0 à 0,21x10⁻³ mol/L ou 0 à 0,21 mM) ou même 0 à 50 mg.L⁻¹ (0 à 0,52x10⁻³ mol/L, 0 à 0,52 mM) comme limites basses.

Parmi les méthodes mises en œuvre pour l'analyse de concentrations de sulfates contenus dans un liquide, on connaît par exemple celle décrite dans la publication Van Staden, 1987, On-line sulfate monitoring by reversed flow injection analysis and alternating reagent injection, Fresenius Z Anal Chem 326: 754-756 [1]. Néanmoins, la méthode décrite dans ce document n'est pas qualifiée pour l'analyse d'eau de mer. De plus, la limite de détection décrite dans le document est de 30 mg.L⁻¹ (0,31x10⁻³ mol/L ou 0,31 mM), cette valeur est trop élevée pour l'analyse d'une eau de mer nanofiltrée.

Il existe donc un réel besoin d'un dispositif et d'un procédé d'analyse de la quantité de sulfate contenue dans un liquide palliant ces défauts, inconvénients et obstacles de l'art antérieur.

Le document Vuillemin et al, 2009, CHEMINI : A new in situ CHEmical MINIaturized analyzer, Elsevier, Deep-Sea Research I 56(2009) 1391-1399 [2] décrit un dispositif et un procédé d'analyse de d'ions fers dissouts (Fe(II) ou Fe(II+III) et de sulfides totaux (H₂S+HS+S²⁻). Il comprend une boucle d'injection (« injection volume ») en spirale, ainsi que deux boucles de réductions (« réduction coil ») en spirales, deux boucles de réaction (« reaction coil ») en spirales, et un spectrophotomètre. Des vannes sont disposées sur ce dispositif pour gérer les flux des différents liquides susceptibles de le traverser.

Le document GB1175967 (Marchon Products LTD) décrit un dispositif et un procédé d'analyse de sulfate au moyen de chlorure de baryum où de nombreux flux de fluide corrosif doivent être contrôlés simultanément. Ce document décrit en particulier un dispositif d'échantillonnage et de dosage des sulfates permettant de prélever un échantillon de fluide à doser et de doser les sulfates dans l'échantillon par turbidimétrie après mélange et réaction avec du chlorure de baryum.

### Exposé de l'invention

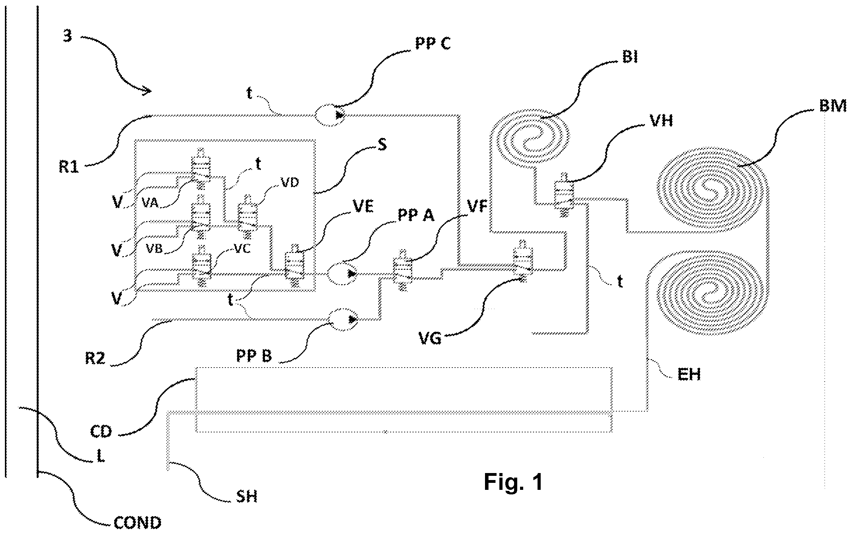

L'invention est définie par les revendications indépendantes 1, 9 et 15. La présente invention permet précisément de résoudre l'ensemble des problèmes techniques et inconvénients de l'art antérieur en fournissant notamment un dispositif (figure 1 annexée) (1) d'analyse de sulfates dans un liquide (L), ledit dispositif comprenant les éléments suivants :
**a)** un circuit analytique (3) étanche comprenant une boucle d'injection (BI) de volume défini connectée à au moins une boucle de mélange (BM), et un moyen d'analyse par turbidimétrie (CD), la boucle d'injection, la boucle de mélange et le moyen d'analyse pouvant être parcourus successivement en continu par un liquide, la boucle d'injection et la boucle de mélange étant séparables par une vanne (VH) ;
**b)** un moyen d'injection (IL) dudit liquide dans ladite boucle d'injection, ledit moyen d'injection comprenant une pompe péristaltique (PPA), un système de vannes (VF, VG) placées entre la pompe (PPA) et ladite boucle d'injection, et un moyen de prélèvement dudit liquide à partir d'une source (S) ;
**c)** un moyen d'injection (ISR), dans ladite boucle d'injection, d'une solution de mise en évidence de sulfates dans ledit liquide par turbidimétrie ; ledit moyen d'injection (ISR) comprenant une pompe péristaltique (PPC) d'injection de ladite solution de mise en évidence de sulfates et une vanne (VG) placée entre ladite pompe (PPC) et ladite boucle d'injection ; et
**d)** un moyen d'injection (ISP) d'une solution de rinçage dudit circuit analytique, ledit moyen d'injection (ISP) comprenant une pompe péristaltique (PPB) d'injection de ladite solution de rinçage et un système de vannes (VF, VG) placées entre la pompe (PPB) et ladite boucle d'injection.

Le dispositif de la présente invention a été nommé par les inventeurs « CHEMINI Sulfates », pour « CHemical MINIaturized analyser Sulfates ». Il s'agit d'un dispositif ou instrument miniaturisé développé pour la mesure en ligne de sulfates dans un liquide. C'est un analyseur chimique qui présente notamment l'avantage d'être utilisable *in situ,* il est basé sur une injection en flux inversé (ou « rFIA » pour « reversed flow injection analysis ») et une détection turbidimétrique. Le CHEMINI Sulfates a pour objectif de mesurer la concentration en sulfates dans un liquide, notamment à la sortie de modules de nanofiltration pour contrôler l'efficacité de ces derniers et déclencher si nécessaire les interventions utiles pour leur maintenance. Il permet avantageusement de répondre à l'ensemble des besoins spécifiques de l'analyse des sulfates pour l'industrie pétrolière.

Par « liquide » on entend tout liquide pour lequel il existe un intérêt à quantifier la quantité de sulfates contenue dans ledit liquide. Il peut s'agir par exemple d'un effluent, par exemple d'eau de mer nanofiltrée ou non. Par exemple, il peut s'agir d'eau de mer nanofiltrée utilisée dans le cas d'exploitation de gisements naturels, par exemple de pétrole, par exemple lors de l'extraction de pétrole offshore, où le procédé de la présente invention est particulièrement avantageux. Il peut s'agir également de mesures en ligne de sulfates dans des eaux de procédés industriels, par exemple pour la fabrication de papier et/ou de cellulose, pour la fabrication d'huiles comestibles, dans des procédés de tanneries, pour réaliser de la surveillance environnementale, dans le traitement des eaux, potables ou non, etc.

En outre, le dispositif de la présente invention se trouve très avantageux du point de vue écologique et économique, car il permet d'économiser et d'optimiser les moyens humains, de matériel, d'opérations de maintenance et de réactifs utilisés. Il présente également un intérêt industriel, car il permet d'augmenter la production du fait de la limitation des opérations de maintenance. Il présente également un intérêt dans le domaine de l'analyse en laboratoire lorsque des analyses sur flux continu sont nécessaires.

Dans le dispositif de la présente invention, le circuit analytique est étanche. Par « étanche » on entend imperméable aux liquides et aux gaz, y compris sous pression interne ou externe. L'étanchéité du dispositif peut être assurée par un choix approprié des matériaux le constituant et par l'utilisation de joints ou soudures étanches appropriés et/ou par confinement d'une partie ou de l'ensemble du dispositif dans un contenant étanche et/ou dans un liquide sous équipression, par exemple de l'huile, par exemple de l'huile diélectrique.

L'étanchéité peut également être assurée par la disposition ou confinement d'une partie ou de l'ensemble du dispositif dans un caisson étanche, qui peut être plastique, verre ou métallique, par exemple en titane. Les matériaux utilisés pour former le dispositif de l'invention, seront aisément choisis par l'homme du métier afin que cette étanchéité soit assurée. Les exemples ci-dessous présentent des exemples de confinement du dispositif de la présente invention.

Selon l'invention, la boucle d'injection possède un volume défini permettant d'injecter une quantité définie de solution de mise en évidence de sulfates présents dans le liquide à analyser par turbidimétrie. Selon l'invention, la boucle d'injection peut disposer par exemple d'un diamètre interne de 0,20 à 1 mm, par exemple de 0,60 à 1 mm, par exemple de 0,80 millimètre. La longueur de la boucle d'injection peut être par exemple de 3 à 10 cm, par exemple de 4 à 8 cm, par exemple de 6 centimètres.

La boucle d'injection peut être de toute forme adaptée à la mise en œuvre de l'invention, par exemple ondulé ou en spirale, de préférence enroulée en spirale, ce qui permet d'obtenir un dispositif compact.

Dans le dispositif de la présente invention, la boucle d'injection est connectée à au moins une boucle de mélange. Cette connexion est de préférence étanche et peut être séparée par une vanne (VH), le liquide à analyser pouvant parcourir en continu la boucle d'injection puis la boucle de mélange.

Selon l'invention, la boucle de mélange peut disposer par exemple d'un diamètre interne identique ou différent de celui de la boucle d'injection, par exemple de 0,20 à 1 mm, par exemple de 0,60 à 1 mm, par exemple de 0,80 millimètre. La longueur de la boucle de mélange peut être par exemple de 1 à 3 m, par exemple de 1,5 à 2,5 m, par exemple de 2 m.

La boucle de mélange peut être de toute forme adaptée à la mise en œuvre de l'invention, par exemple ondulé ou en spirale, de préférence enroulée en spirale, ce qui permet d'obtenir un dispositif compact. Selon l'invention, le dispositif peut comprendre une ou plusieurs boucles de mélanges, l'une à la suite de l'autre, par exemple deux, trois ou quatre boucles de mélange, de préférence deux boucles de mélange, qui se suivent et qui peuvent être parcourue en continu par le liquide à analyser et par la solution de mise en évidence des sulfates pour leur mélange.

La boucle d'injection et la boucle de mélange peuvent être constituées d'un matériau identique ou différent, de préférence identique, de préférence d'un matériau n'interagissant pas avec le liquide et la solution de mise en évidence des sulfates, et pouvant également, suivant les conditions d'utilisation, devoir résister à des pressions internes et/ou externes supérieures à la pression atmosphérique. Il peut s'agir par exemple d'un métal choisi dans le groupe comprenant de l'inox ou d'un plastique type PEEK. Avantageusement, le métal peut être recouvert sur sa surface interne par du Téflon (marque déposée) ou tout autre matériau connu de l'homme du métier et compatible avec le liquide à analyser, les sulfates et les réactifs utilisés.

La boucle de mélange permet un mélange du liquide à analyser et de la solution de mise en évidence des sulfates éventuellement présents dans ledit liquide, avant que ce mélange passe dans le moyen d'analyse par turbidimétrie.

Le moyen d'analyse par turbidimétrie permet de mettre en évidence les sulfates éventuellement présents dans le liquide à analyser, et de les quantifier le cas échéant, grâce à l'interaction desdits sulfates avec la solution de mise en évidence de ceux-ci. Ce moyen d'analyse peut être composé d'une cellule de détection (DC), d'une carte électronique de détection (CE), de LEDs ou de micro-LEDs, et d'une photodiode. Ce dispositif présente avantageusement 3 LEDs aux longueurs d'onde de 520nm (+/- 30 nm) qui est la longueur d'onde de détection du sulfate de baryum, 650nm (+/- 20 nm) et 810nm (+/- 35nm).

La partie détection de l'analyseur peut être composée par exemple d'une cellule de mesure, qui peut avoir par exemple une longueur de 2 à 4 cm, et par exemple un diamètre interne de 0,5 à 1,5 mm, par exemple de 1 mm, fabriquée par la société HELLMA (Allemagne). Elle peut comprendre une carte électronique de détection. La carte électronique pour la détection est par exemple une carte capable d'accueillir trois LEDs et une photodiode. La lumière émise par les LEDs est transférée à la cellule de mesure puis revient au détecteur de type photodiode par des fibres optiques, par exemple de PMMA, avec par exemple un diamètre interne de 1 mm.

Le précipité de sulfate de baryum atténue la lumière incidente de la cellule de détection. La lumière résiduelle sortant de la cellule de détection peut alors être captée par une photodiode.

La cellule de détection est traversée par le circuit hydraulique. Le tuyau peut être branché sur la cellule à l'aide de connectiques par exemple de type Minstac (marque déposée) (Société Lee Company, Etats-Unis), le tuyau en sortie peut être par exemple relié à un contenant d'accueil du liquide analysé (non représenté).

Le moyen d'analyse permet ainsi de détecter par turbidimétrie une concentration de sulfates dans ledit liquide analysé par formation du précipité dans la boucle de mélange, par exemple de BaSO₄.

Dans la présente invention, le terme « vanne » désigne un moyen servant à arrêter le liquide ou la solution dans le dispositif de la présente invention. Selon l'invention, les vannes peuvent être toute vanne connue de l'homme du métier et adaptée au dispositif de la présente invention. Il peut s'agir par exemple d'électrovannes. Parmi les vannes utilisées, on connaît par exemple celles décrites dans la publication Vuillemin et al, 2009, CHEMINI : A new in situ CHEmical MINIaturized analyzer, Elsevier, Deep-Sea Research I 56(2009) 1391-1399 **[2]**. Selon l'invention, les vannes peuvent être des vannes solénoïdes, par exemple trois voies (VA à VH).

Les « pompes péristaltiques » permettent d'injecter le liquide et les solutions de mise en évidence et de rinçage dans le dispositif de la présente invention. Parmi les pompes utilisées, on connaît celles décrites dans la publication Vuillemin et al, 2009 **[2].** Les pompes péristaltiques peuvent être par exemple monocanales (PPA, PPB et PPC).

Des actionneurs peuvent être utilisés pour la circulation des différents fluides dans le dispositif : solution de mise en évidence, solution de rinçage, étalons et liquide à analyser.

Selon l'invention, la source (S) peut comprendre au moins une voie (V), par exemple (V1), (V2), (V3), (V4), (V5), (V6), de prélèvement du liquide circulant ou en mouvement, ladite voie comprenant au moins deux vannes (VA, VB, VC, VD, VE) placées entre ledit liquide en mouvement et la pompe péristaltique (PPA).

Par « voie (V) de prélèvement », on entend un moyen permettant de connecter le dispositif de la présente invention à un conduit dans lequel le liquide à analyser circule, par exemple à un module de nanofiltration d'eau de mer ou un circuit d'injection d'eau de mer nanofiltrée dans des réservoirs naturels, par exemple pétroliers, par exemple offshore, l'eau de mer constituant ledit liquide (L) en mouvement. Tout moyen de connexion connu de l'Homme du métier pour prélever un liquide dans un premier circuit et l'amener dans un circuit d'analyse peut être utilisé.

Le module de nanofiltration duquel on souhaite vérifier la qualité de filtration des sulfates peut être par exemple un dispositif de traitement de désulfatation constitué de modules membranaires et dont le suivi de la teneur en sulfates peut être réalisé sur le collecteur de sortie de l'ensemble des modules.

Selon l'invention, le dispositif peut comprendre un réservoir de solution de mise en évidence des sulfates duquel la pompe péristaltique (PPC) prélève ladite solution pour l'injecter dans le circuit analytique. Il peut s'agir d'une poche de transfert en plastique souple en polyéthylène ou équivalent ou flaconnage au moyen de flacons en verre.

Selon l'invention, le dispositif peut comprendre un réservoir de solution de rinçage duquel la pompe péristaltique (PPB) prélève ladite solution pour l'injecter dans le circuit analytique pour son rinçage. Il peut s'agir d'une poche de transfert.

Les connexions entre tubes et vannes se font au moyen de connecteurs MINSTAC (marque de commerce) (société Lee Co) et de connecteurs Lueurs (marque de commerce) (société Fisher).

Selon l'invention, le dispositif de la présente invention peut être automatisé, par exemple par des moyens de programmation et de contrôle d'injection en flux continu inversé, dans ladite boucle d'injection, de la solution de mise en évidence de sulfates dans ledit liquide, ladite solution pouvant comprendre par exemple une quantité stœchiométrique de BaCl₂ ou d'un composé équivalent connu de l'Homme du métier permettant de détecter une concentration maximale de sulfates acceptables dans ledit liquide.

Selon l'invention, ces moyens de contrôle sont connectés par exemple aux vannes (VA, VB, VC, VD, VE, VF, VG et VH), aux pompes péristaltiques (PPA, PPB et PPC) et au moyen d'analyse et permettent d'automatiser le dispositif de la présente invention, par exemple pour un contrôle automatisé du taux de sulfates en sortie de modules de nanofiltration d'eau de mer de l'industrie pétrolière offshore ou de tout autre installation nécessitant un contrôle de la concentration de sulfate dans un effluent liquide.

Ce contrôle peut être réalisé par exemple au moyen d'une carte dessinée et conçue sur la base d'un microcontrôleur ATMEL Atmega (8 MHz, 3,3 V). Cette carte présente de préférence les caractéristiques suivantes : 64 ko de RAM, 512 ko de data Flash, 128 ko disponible pour le code, bus SPI et I2C, 3 timers, 8 canaux ADC, 2 UART, 15 ports I/O et des dimensions de 55 x 55 mm. Cette carte permet de gérer chaque composant du dispositif de l'invention en détail ainsi que le module optique. La carte basée sur un microcontrôleur permet le contrôle du module optique, des éléments de l'appareil (électrovannes, pompes) ainsi que la communication avec l'IHM d'un ordinateur.

Le dispositif de la présente invention peut comprendre en outre un logiciel d'exploitation Interface Homme-Machine (IHM), par exemple développé sous Visual Basic ou tout autre système permettant de commander les différents éléments de l'appareil et d'exporter les données obtenues, par exemple vers un logiciel de calculs de type Excel. Les caractéristiques du logiciel d'exploitation IHM et logiciel embarqué CHEMINI ont été déposées à l'Agence pour la Protection des Programmes sous la référence: IDDN.FR.001.430027.000.R.P.2009.000.30625 (certificat de dépôt daté du 21 octobre 2009).

Ainsi, selon l'invention, le dispositif peut être programmé et contrôlé au moyen d'une interface homme machine (IHM). Selon l'invention, ce contrôle peut par exemple être tel qu'il peut être exécuté en trois niveaux : un mode bas-niveau, un mode de contrôle à distance et un mode endurance autonome. En mode bas-niveau, l'utilisateur a accès à chaque composant du dispositif de l'invention et chaque paramètre manuellement. Il peut modifier la vitesse de rotation d'une pompe péristaltique en particulier ou commuter ou non chaque vanne. Ce mode peut être utilisé pour les opérations de maintenance et de développements primaires. Dans le mode de contrôle à distance de l'analyseur, l'utilisateur peut lancer une programmation préenregistrée pour la mise en œuvre du dispositif de la présente invention. Ce mode peut être utilisé pour les acquisitions d'une mesure sur le court terme ou lors d'un accès assez simple à l'analyseur. Enfin, le mode de fonctionnement autonome permet de lancer une sélection de programmes, appelé également cycles, préenregistrés. Cette fonction est utile pour l'acquisition de longues séries de mesures ou pour des fonctionnements autonomes à heure fixe sur plusieurs jours par exemple. Les données peuvent être stockées dans une mémoire interne du dispositif de la présente invention et peuvent être extraites en utilisant le logiciel d'exploitation de l'analyseur (IHM). Le traitement des données brutes peut également être effectué par le logiciel pour obtenir la valeur de l'intensité lumineuse absorbée par les particules (turbidimétrie) et minimiser l'effet du bruit des mesures. Les données brutes et traitées peuvent ensuite être transférées à partir du logiciel IHM sur un logiciel de calcul EXCEL pour approfondir le traitement des données.

Le dispositif peut comprendre en effet une mémoire permettant de mémoriser des analyses successives. Il peut s'agir par exemple d'une mémoire de type Flash SPI AT45DB321, qui peut être par exemple d'une capacité de 32 Mégabits ou plus.

Selon l'invention, le dispositif peut par exemple dans son ensemble, c'est-à-dire son module hydraulique comprenant le circuit d'injection et le circuit de mélange, son module optique, comprenant le moyen d'analyse, et fonctionner en 12 V.

Selon l'invention, le dispositif peut communiquer avec le logiciel IHM par exemple par une liaison série RS-232.

Selon l'invention, le dispositif peut être confiné au sein d'au moins une enceinte étanche d'immersion sous-marine.

Il peut s'agir par exemple d'une seule enceinte étanche. Cette version est notamment adaptée à une utilisation côtière du dispositif de l'invention. Il peut s'agir par exemple d'une enceinte immergeable à 10 mètres ou plus suivant les matériaux utilisés et l'étanchéité de ceux-ci. Cette enceinte peut être en inox d'une épaisseur de 1,5 mm ou en polyméthacrylate de méthyle (PMMA) d'une épaisseur de 10 mm. Les dimensions de cette enceinte sont adaptées pour recevoir le dispositif de l'invention, par exemple de 20 à 25 cm de haut, par exemple de 12 à 16 cm de longueur et de 10 à 14 cm de largeur pour un dispositif défini pour s'y insérer.

Selon l'invention, le dispositif peut être composé de deux parties ou blocs distincts, l'une comprenant les boucles d'injection et de mélange ou module hydraulique, et l'autre comprenant le moyen d'analyse par turbidimétrie ou module optique et l'électronique. Cette version peut être utile comme version « grands fonds » (DVGF), par exemple immergeable jusqu'à 6000 mètres. Dans ce cas, le module hydraulique est de préférence en équipression, par exemple dans de l'huile diélectrique, par exemple Fluorinert, FC77, 3M (marque de commerce) (société 3M, USA), avec un confinement en caisson dont le couvercle supérieur peut être en PMMA transparent, le couvercle inférieur peut être en polychlorure de vinyle (PVC) par exemple de 15mm d'épaisseur, et le corps du module peut être par exemple en titane T40, par exemple avec une épaisseur de 1,5mm. Le module optique/électronique, le couvercle supérieur, le corps et le fond peuvent être en alliage de titane TA6V avec les dimensions pour le couvercle supérieur par exemple 32mm d'épaisseur, pour le corps par exemple 11mm d'épaisseur et pour le fond par exemple 25 mm d'épaisseur.

Le module optique / électronique peut être confiné par exemple dans un caisson étanche en titane. Les dimensions de ces enceintes sont adaptées pour recevoir le dispositif de l'invention en deux parties, par exemple de 25 à 30 cm de haut, par exemple de 12 à 16 cm de diamètre pour le confinement du module optique/électronique et par exemple de 12 à 14 cm de haut, et par exemple de 13 à 16 cm de longueur et de 10 à 14 cm de largeur pour le module hydraulique pour un dispositif défini pour s'y insérer.

La présente invention se rapporte également à l'utilisation du dispositif de l'invention pour l'analyse de sulfates dans un liquide comme défini ci-dessus, par exemple dans une eau de mer, par exemple nanofiltrée.

Par exemple, dans ce dernier cas, le liquide (L) représente ladite eau de mer nanofiltrée. Selon l'invention, cette eau de mer nanofiltrée peut être l'eau de mer qui est destinée à être injectée dans un réservoir naturel ou une cavité pour extraire du pétrole, une voie de prélèvement (V) pouvant permettre d'échantillonner *in situ* cette eau de mer nanofiltrée pour en analyser son contenu en sulfates.

Par exemple, selon l'invention, quelque soit le liquide, ladite utilisation peut comprendre les étapes suivantes :
a) injection en flux continu inversé, dans ladite boucle d'injection, d'une solution de mise en évidence de sulfates dans ledit liquide, ladite solution comprenant une quantité stœchiométrique de BaCl₂ ou d'un composant permettant de détecter une concentration maximale de sulfates acceptable dans ledit liquide par formation de BaSO₄, ou d'un sulfate de cet autre composant, ladite solution de mise en évidence pouvant comprendre en outre des cristaux de thymol et de la gélatine ;
b) injection dudit liquide (L) à analyser dans ladite boucle d'injection, ledit liquide à analyser poussant ladite solution injectée à l'étape a) et se mélangeant à celle-ci dans la boucle de mélange ; et
c) mesure par turbidimétrie du BaSO₄, ou du sulfate de l'autre composant, formé le cas échéant lors de l'étape b) via le moyen d'analyse par turbidimétrie (CD).

Avantageusement, l'étape a) peut être précédée d'une étape de purge du circuit au moyen du liquide (L) en le faisant circuler grâce à la pompe péristaltique (PPA) dans les boucles d'injection et de mélange et dans le moyen d'analyse par turbidimétrie. Cette étape de purge permet avantageusement d'éliminer tout résidu d'une analyse précédente et de placer le dispositif dans des conditions optimales pour la nouvelle analyse.

Avantageusement l'étape c) de mesure peut être suivie d'une étape de rinçage du circuit analytique par injection dans celui-ci d'une solution de rinçage éliminant le BaSO₄ formé le cas échéant, ou le sulfate de l'autre composant, ladite solution de rinçage pouvant comprendre par exemple de l'éthylène diamine tétra-acétate (EDTA) ou un produit équivalent connu de l'homme du métier permettant d'éliminer le BaSO₄ encore présent dans le circuit analytique. En effet, lorsqu'on utilise du BaCl₂ pour détecter les sulfates, en présence de baryum (Ba²⁺), ces derniers réagissent pour former un précipité de sulfate de baryum (BaSO₄). L'ion baryum Ba²⁺ de la solution de mise en évidence, ou réactif, réagit avec l'ion sulfate SO₄²⁻ du liquide, ou échantillon, pour former un précipité de sulfate de baryum BaSO₄ détectable par turbidimétrie à 520 nm.

Ce précipité peut toutefois à la longue colmater le circuit analytique, ce qui peut engendrer une diminution de la précision sur les mesures, une baisse d'efficacité et une dégradation du matériel d'analyse. L'injection d'une solution de rinçage, par exemple d'une solution alcaline d'EDTA, permet de dissoudre tout précipité formé et ainsi d'empêcher le colmatage du circuit.

Cette étape de rinçage peut être à nouveau suivie d'une étape de purge comme définie précédemment, avant d'opérer à une nouvelle analyse.

Selon l'invention, la solution de mise en évidence peut être une solution comprenant du BaCl₂ ou une solution comprenant un autre composant connu de l'Homme du métier formant un sulfate détectable par turbidimétrie ou, de manière plus générale, un composé sulfaté détectable par les moyens connus de l'Homme du métier permettant de détecter des sulfates. Il peut s'agir par exemple d'une solution d'ions Fe³⁺ par exemple lorsqu'on utilise une méthode spectrocolorimétrique conduisant à la formation du composé FeSO₄⁺ détecté entre 320 et 360 nm, ou encore d'une solution de chromate de baryum BaCrO₄ par exemple lorsqu'on utilise une méthode spectrocolorimétrique avec utilisation d'une résine échangeuse d'ion conduisant à la libération des ions chromates CrO₄²⁻ dont l'absorbance est mesurée à 370 nm.

Selon l'invention, avantageusement, la concentration en BaCl₂ ou en composant connu de l'Homme du métier est une concentration stœchiométrique permettant de détecter une concentration maximale de sulfates acceptable dans ledit liquide par formation de BaSO₄, ou d'un sulfate de cet autre composant. Ainsi, si l'usage du liquide ou l'installation dans laquelle circule le liquide ou l'opérateur exige que la concentration en sulfates du liquide à analyser ne doit pas dépasser 40 mg.L⁻¹ (0,42x10⁻³ mol/L ou 0,42 mM), on utilisera de préférence une quantité stœchiométrique de BaCl₂ ou composant similaire permettant grâce au dispositif de la présente invention et à son utilisation de détecter ce seuil maximum. Ainsi, selon l'invention, la quantité de réactif utilisée est optimisée, limitant l'impact écologique, et la quantité de précipité formée dans le circuit analytique est minimale, limitant les opérations de maintenance de ce circuit. Cette optimisation vient en plus de celle obtenue grâce au dispositif de la présente invention lui-même, notamment grâce au flux inversé rFIA.

En outre, selon l'invention, l'ajout combiné de cristaux de thymol et de gélatine permet de laisser en suspension les particules et donc d'éviter les dépôts sur les parois des tubes et dans la cellule de mesure. Les cristaux de thymol peuvent être ajoutés à raison de 0,05 à 0,2 g/L de solution. La gélatine peut être ajoutée à raison de 0,5 à 3 g/L.

Par exemple, selon l'invention, on peut utiliser le cycle suivant : dans un premier temps, le liquide (L) est introduit dans le circuit analytique entrainé par la pompe péristaltique (PPA), pendant une durée suffisante pour purger le circuit. Cette durée peut être de 1 à 5 minutes par exemple ou de tout autre durée que l'opérateur estime appropriée. Dans un second temps, la solution de mise en évidence des sulfates, par exemple une solution de BaCl₂, est introduite dans la boucle d'injection possédant un volume prédéfini propulsée par la pompe péristaltique (PPC). Enfin, le liquide est remis en circulation par la pompe péristaltique (PPA) dans la boucle d'injection, ledit liquide poussant cette fois-ci la solution de mise en évidence des sulfates, par exemple BaCl₂, présente dans la boucle d'injection vers la boucle de mélange où le liquide et la solution de mise en évidence se mélangent. Par ce mélange, si des sulfates sont présents dans le liquide, ils réagissent par exemple avec BaCl₂ pour former un précipité de BaSO₄, ce précipité passant devant la cellule de détection où il est détecté et mesuré. A la fin du cycle, on peut procéder à un rinçage, comme décrit ci-dessus, en injectant la solution de rinçage au moyen de la pompe péristaltique (PPB). Le dispositif est ainsi à nouveau prêt pour un nouveau cycle d'analyse. Après injection du réactif de mise en évidence des sulfates, il est possible de procéder à un arrêt du flux (également appelé Stop Flow). Ceci laisse plus de temps à la réaction chimique d'avoir lieu et permet ainsi d'obtenir une meilleure définition du signal en sortie. Cette option est particulièrement intéressante en cas de faibles concentrations en sulfates.

Selon l'invention, on peut procéder avant la mise en œuvre du procédé de la présente invention à un étalonnage du dispositif de l'invention au moyen d'un ou de plusieurs standard(s) préparé(s) avec de l'eau de mer artificielle et des concentrations choisies en sulfates. Différentes mesures de turbidimétrie sont réalisées en utilisant le dispositif de la présente invention, par exemple à 520 nm lorsque du BaCl₂ est utilisé pour la solution de mise en évidence des sulfates. Par ce biais de l'eau de mer artificielle, la matrice aqueuse des standards, ou étalons, est la plus fidèle possible à l'eau de mer tout en étant sans sulfates ou avec des concentrations déterminées.

La gamme de mesure exploitable grâce au procédé de la présente invention et à son utilisation est de 0 à 100 mg de sulfates / L avec une limite de détection de 4 mg/L (0,04x10⁻³ mol/L ou 0,04 mM ou 40 µM), ce qui est remarquable.

Le dispositif de la présente invention permet de s'intégrer parfaitement dans une installation pétrolière offshore existante, alors que les moyens de l'art antérieurs ne sont tout simplement pas appropriés aux besoins de l'industrie pétrolière.

Le dispositif de la présente invention, permet en outre, de par sa structure, de réaliser des analyses chimiques *in situ* automatisées, et ce dans de larges gammes de température et de pression, par exemple entre 4 et 35 °C, et par exemple entre 1.10⁵ et 600.10⁵ Pa (pour la version grand fond). Pour la version côtière, la pression maximale supportée est de 2.10⁵ Pa en opérationnel. Ainsi qu'un étalonnage in situ de l'analyseur au moyen d'étalons « embarqués ».

Ce dispositif de la présente invention permet en outre d'obtenir des valeurs de concentration en temps réel et ainsi de détecter rapidement des anomalies dans un dispositif, par exemple de traitement d'effluents liquides, provoquant un taux de sulfates trop élevé dans un liquide, par exemple pour une injection dans des réservoirs de gisements naturels, par exemple pétroliers, ou tout autre procédé industriel, par exemple ceux précités. Il permet une mesure en ligne des sulfates dans une matrice aussi complexe que de l'eau de mer. Le dispositif de la présente invention et son utilisation ne nécessitent pas l'utilisation de colonnes échangeuses d'ions avant analyse et la calibration du dispositif peut être réalisée avec des standards préparés à partir d'eau de mer artificielle.

Le dispositif de la présente invention permet également une faible consommation d'énergie et de réactifs chimiques. La quantité en réactifs utilisée par exemple en offshore pétrolier est grâce à la présente invention plus limitée, notamment grâce au flux inversé rFIA. Cette consommation en réactifs minimale permet avantageusement de limiter les opérations de maintenance sur site et de fournir une autonomie de longue durée.

Le dispositif de la présente invention présente également l'avantage de ne pas nécessiter de personnel sur site régulièrement. L'analyseur peut en effet être programmé, au moyen d'un programmateur, sur de longues périodes avec une fréquence d'analyse définie par les utilisateurs. La seule maintenance concerne le changement des réactifs qui peut s'effectuer qu'au bout de plusieurs mois, suivant notamment le volume de réservoir de réactif prévu pour la solution de mise en évidence des sulfates.

D'autres avantages pourront encore apparaître à l'homme du métier à la lecture des exemples ci-dessous, illustrés par les figures annexées, donnés à titre illustratif et non limitatif.

### Brève description des figures

- La figure 1 représente schématiquement un dispositif selon l'invention.
- La figure 2 représente une courbe d'étalonnage d'analyse turbidimétrique à 420 nm, établie à partir d'étalons de concentrations de sulfates en eau de mer artificielle. En abscisse est représentée la concentration (C) en sulfates (mg.L⁻¹) et en ordonnée l'intensité (I) mesurée.
- La figure 3 représente une courbe d'étalonnage d'analyse turbidimétrique à 810 nm, établie à partir d'étalons de concentrations de sulfates en eau de mer artificielle. En abscisse est représentée la concentration (C) en sulfates (mg.L⁻¹) et en ordonnée l'intensité (I) mesurée.
- La figure 4a représente des courbes d'étalonnages d'analyse turbidimétrique à 520 nm (1 GE et 2 GE) établies à partir d'étalons de concentrations de sulfates en eau de mer artificielle, pour des concentrations en sulfates comprises entre 0 et 60 mg/L.
   En abscisse de la courbe 1GE est représenté l'index des mesures (une mesure d'absorbance est réalisée toutes les 250 ms) et en ordonnée est représentée l'absorbance (A) mesurée (en milliers). En abscisse de la courbe 2 GE est représenté la concentration (C) en sulfates (mg.L⁻¹) et en ordonnée l'intensité (I) mesurée. La courbe 2 GE a pour équation y = 7663,4x + 125185 (R² = 1)

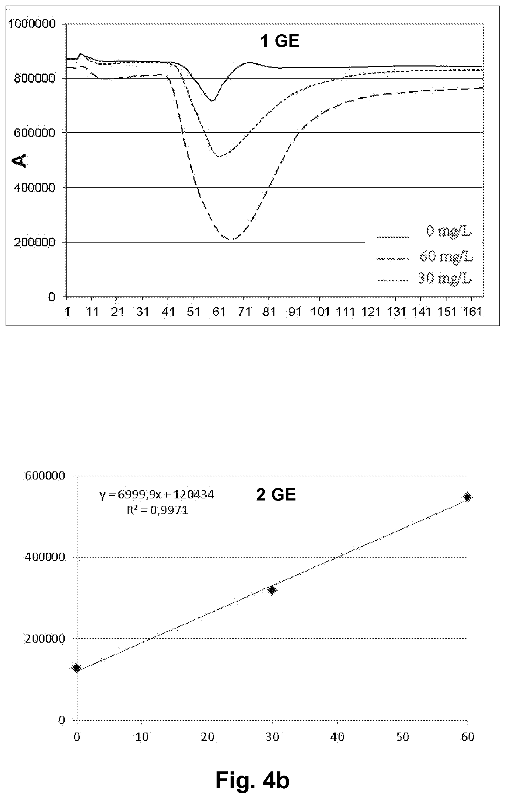

- La figure 4b représente des courbes d'étalonnages d'analyse turbidimétrique à 520 nm (1 GE et 2 GE) établies à partir d'étalons de concentrations de sulfates en eau de mer artificielle. pour des concentrations en sulfates comprises entre 0 et 60 mg/L.
   En abscisse de la courbe 1GE est représenté l'index des mesures (une mesure d'absorbance est réalisée toutes les 250 ms) et en ordonnée est représentée l'absorbance (A) mesurée (en milliers). En abscisse de la courbe 2 GE est représentée la concentration (C) en sulfates (mg.L⁻¹) et en ordonnée l'intensité (I) mesurée. La courbe 2 GE a pour équation y = 6999,9x + 120434 (R² = 0,9971).

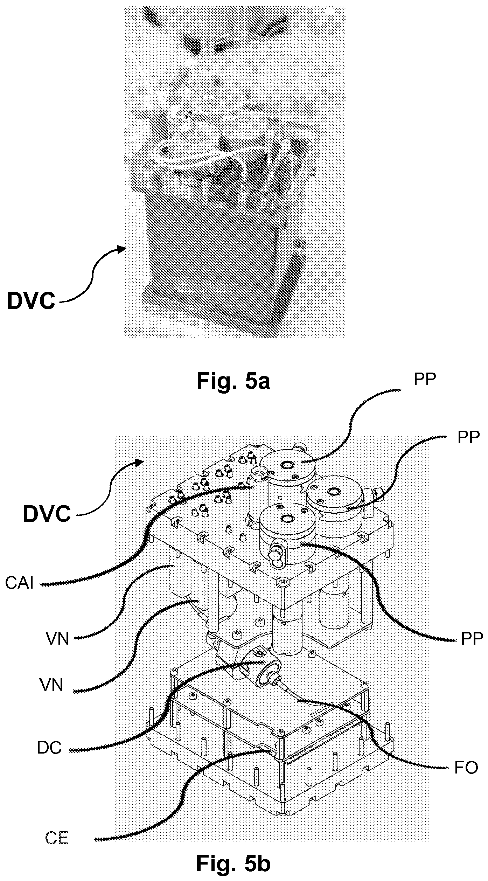

- La figure 5a est une photographie d'un dispositif selon l'invention, confiné, version côtier.
- La figure 5b est une représentation schématique éclatée du dispositif représenté sur la figure 5a.

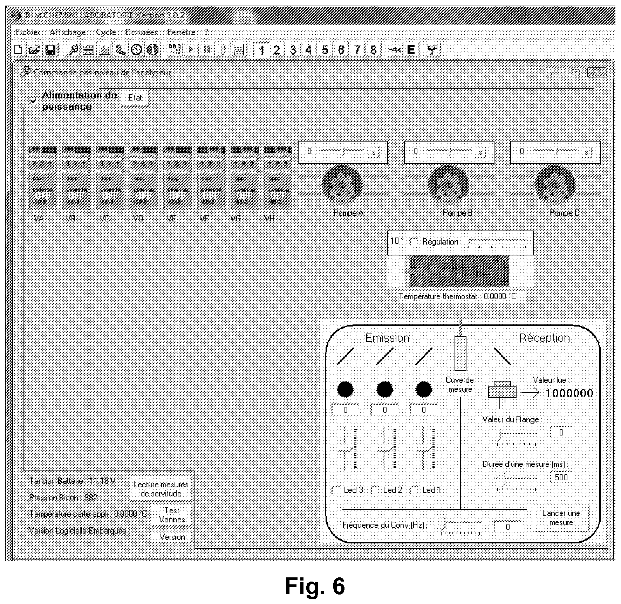

- La figure 6 est une copie écran d'une interface homme machine (IHM) utilisée dans un exemple de mise en œuvre de la présente invention.

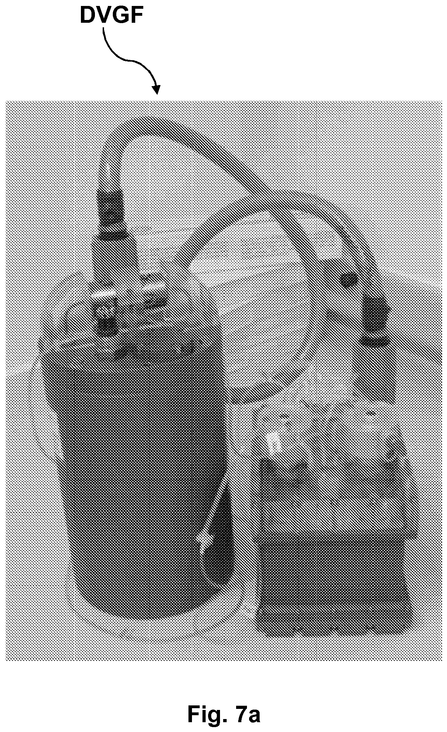

- La figure 7a est une photographie d'un dispositif selon l'invention, confiné, version grands fonds.

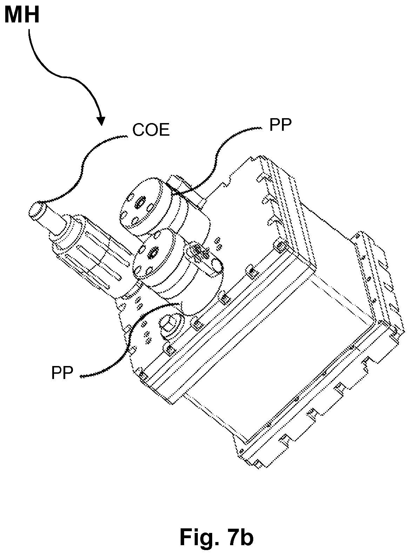

- La figure 7b représente schématiquement le module hydraulique du dispositif version grands fonds représenté sur la photographie de la figure 7a.

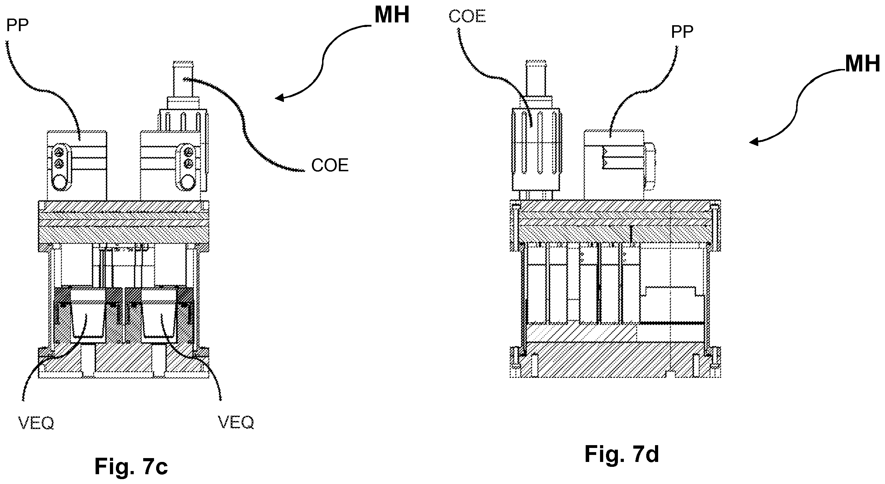

- La figure 7c et la figure 7d représentent schématiquement un module hydraulique d'un dispositif version grand fonds. Sur la figure 7c sont représentées schématiquement des vessies d'équipression (VEQ).

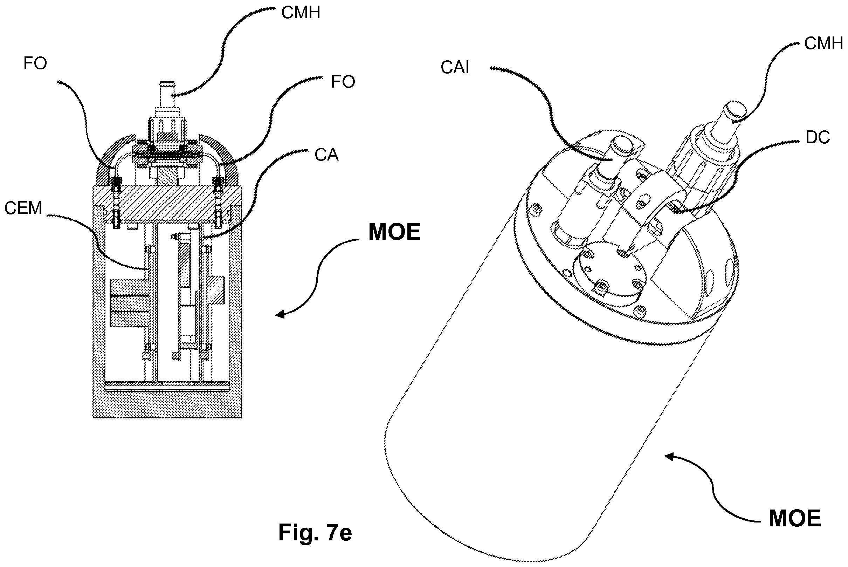

- La figure 7e représente schématiquement un module optique/électronique d'un dispositif version grand fonds.

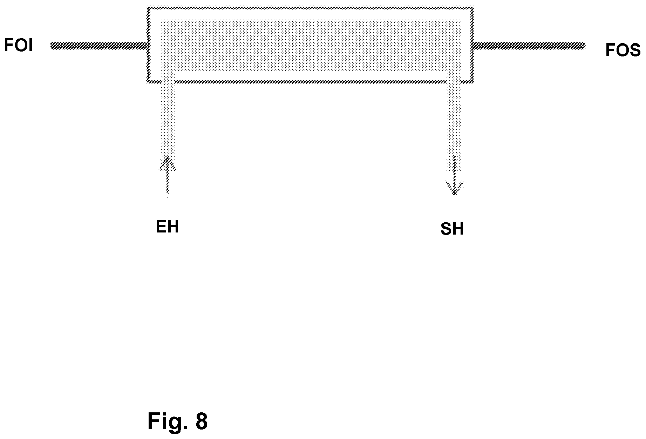

- La figure 8 représente schématiquement la connexion entre la cellule de détection et le circuit hydraulique.

### EXEMPLES

### Exemple 1 : dispositif d'analyse selon l'invention

Un exemple de dispositif d'analyse selon la présente invention est représenté schématiquement sur la figure 1.

Le dispositif comprend un circuit analytique (3) étanche comprenant une boucle d'injection (BI) double de volume défini connectée à au moins une boucle de mélange (BM), et un moyen d'analyse par turbidimétrie (CD), la boucle d'injection, la boucle de mélange et le moyen d'analyse peuvent être parcourus en continu par un liquide, la boucle d'injection et la boucle de mélange étant séparés par une vanne (VH).

La boucle d'injection est un tube en Téflon (marque déposée) de forme enroulée, dispose d'un diamètre interne de 0,80 millimètre et d'une longueur d'environ 6 centimètres. Le volume prédéfini de cette boucle d'injection permet d'injecter une quantité définie de solution de mise en évidence des sulfates grâce à la pompe péristaltique (PPC).

La boucle de mélange est également un tube en Téflon (marque déposée) et de forme enroulée. Elle dispose d'un diamètre interne de 0,80 millimètre et une longueur de 2 mètres.

Les boucles d'injection et de mélange sont obtenues par simple enroulement. Les boucles d'injection et de mélange sont connectées entre elles et séparées par des vannes solénoïdes. Les connexions entre tubes et vannes sont assurées par des connecteurs MINSTAC (marque déposée) (Société Lee Company, Etats-Unis) et de connecteurs Lueurs (marque de commerce) (Société Fisher, Suisse).

Le moyen de mesure par turbidimétrie (CD) comprend 3 LEDs, une photodiode, une carte électronique de détection (CE) et une cellule de détection (DC) fabriquée par la société HELLMA (Allemagne). La figure 8 représente schématiquement la cellule de détection utilisée dans cet exemple. Sur cette figure, on voit la cellule de détection qui est traversée par le circuit hydraulique, la fibre optique entrante (FOI) et la fibre optique sortante (FOS). Le tuyau (EH) est branché sur la cellule à l'aide de connectiques de type Minstac (marque déposée) (Société Lee Company, Etats-Unis), le tuyau en sortie (SH) peut être par exemple relié à un contenant d'accueil du liquide analysé (non représenté sur le schéma) d'une longueur de 3 centimètres et de 1 millimètre de diamètre, et une carte électronique (CE) de détection EVOSENS (marque de commerce) (Société EVOSENS, France). La carte électronique pour la détection accueille trois diodes électroluminescentes (LED) EVOSENS (marque de commerce) (Société EVOSENS, France), et une photodiode EVOSENS (marque de commerce) (Société EVOSENS, France). La lumière émise par les diodes électroluminescentes est transférée de la cellule de détection vers le détecteur de type photodiode par des fibres optiques (FO) faites en polyméthacrylate de méthyle (PMMA) et de diamètre interne égal à 1 millimètre. Le tube en Téflon de la boucle de mélange se raccorde à la cellule de détection par un raccord MINSTAC (marque déposée) (Société Lee Company, Etats-Unis).

Le dispositif comprend également un moyen d'injection (IL) dudit liquide dans ladite boucle d'injection, ledit moyen d'injection comprenant une pompe péristaltique (PPA), un système de vannes (VF, VG) placées entre la pompe (PPA) et ladite boucle d'injection, et un moyen de prélèvement dudit liquide à partir d'une source (S).

Il comprend également un moyen d'injection (ISR), dans ladite boucle d'injection, d'une solution de mise en évidence de sulfates dans ledit liquide par turbidimétrie; ledit moyen d'injection (ISR) comprenant une pompe péristaltique (PPC) d'injection de ladite solution de mise en évidence de sulfates et une vanne (VG) est placée entre ladite pompe (PPC) et ladite boucle d'injection.

Le dispositif comprend également un moyen d'injection (ISP) d'une solution de rinçage dudit circuit analytique, ledit moyen d'injection (ISP) comprenant une pompe péristaltique (PPB) d'injection de ladite solution de rinçage et un système de vannes (VF, VG) placées entre la pompe (PPB) et ladite boucle d'injection.

Ainsi, le circuit analytique du dispositif inclut huit vannes solénoïdes (VA à VH) et trois pompes péristaltiques monocanales (PPA à PPC) reliées entre elles par des tuyaux (t) Tygon R3603 (marque de commerce) (société Fisher Scientific, USA) de diamètre interne 1,14 mm).

Les pompes péristaltiques (PP) utilisées sont des pompes monocanal Medorex (marque de commerce) (société Medorex, Allemagne) (OEM Peristaltic-Pump HP/90 42-1-6-1,02x0,8 12 VDC 6VA, 1-channel, 6-rollers). Les vannes (VN) utilisées sont des vannes solénoïdes trois voies (une voie commune, une voie normalement fermée et une voie normalement ouverte) marinisées LVM 105R-6 (marque de commerce) (société SMC, Japon).

Ces pompes et vannes sont utilisées pour la circulation des différents fluides dans le dispositif : réactifs, étalons et liquide à analyser (L). Trois des huit vannes solénoïdes (VF à VH) sont utilisées pour l'injection du réactif dans le flux du liquide (L) à analyser. Les cinq autres vannes (VA à VE) sont utilisées pour la sélection des étalons (Voies V) ou du liquide à analyser (L).

La source (S) comprend six voies (V) de prélèvement du liquide en mouvement à analyser, lesdites voies comprenant des vannes (VA, VB, VC, VD et VE) placées entre ledit liquide en mouvement et la pompe péristaltique (PPA). Les vannes (VA, VB et VC) permettent la connexion à un ou plusieurs conduits (COND) dans lequel (lesquels) circule le liquide à analyser.

Des poches de transfert (R1, R2) sont prévues pour contenir les réactifs chimiques ainsi que les étalons nécessaires à l'étalonnage de l'analyseur. Ces poches de transfert sont connectées à l'appareil via des connecteurs Lueurs (marque de commerce) (Société Fisher, Suisse). Les poches de transfert utilisées pour les étalons ainsi que pour la solution de rinçage sont en polyéthylène (PE), de volume 600 mL ou 1 L. Pour la solution de réactif, l'utilisation d'un contenant en verre est acceptable.

La vanne solénoïde (VH) est reliée à un tuyau (t) d'évacuation des liquides circulants dans le dispositif, utile par exemple pour le rinçage et la purge du dispositif. Ce tuyau peut être relié à un contenant d'accueil du liquide analysé (non représenté).

Le moyen de mesure par turbidimétrie est lui aussi relié à un tuyau (t) d'évacuation des liquides circulants dans le dispositif, utile par exemple pour le rinçage et la purge du dispositif jusqu'au moyen d'analyse. Ce tuyau peut être relié à un contenant d'accueil du liquide analysé (non représenté).

Les vannes, pompes péristaltiques et le moyen de détection sont connectés à des moyens électroniques permettant de faire fonctionner le dispositif et de le contrôler. Cette partie électronique (non représentée) du dispositif est composée d'une carte dessinée et conçue par les inventeurs sur la base d'un microcontrôleur ATMEL Atmega (marque de commerce, USA). Cette carte a permis la gestion de chaque moyen en détail : la vitesse et le sens des pompes, l'alimentation ou non des vannes (on/off), le réglage de la puissance des LEDs, la mesure de la puissance en retour, le stockage des données de mesure, une communication avec l'utilisateur.

L'ensemble du dispositif fonctionne sous 12V.

Un logiciel a été développé par les inventeurs sous Visual Basic, il offre une interface homme machine (IHM) permettant à un opérateur de contrôler à distance le dispositif de l'invention. Ce logiciel permet, au travers de l'interface graphique, d'actionner les différents éléments de l'appareil (pompe, vanne, LED, etc.), de programmer et d'enregistrer des cycles (c'est-à-dire un script de commandes permettant d'automatiser l'enchainement des opérations unitaires nécessaires à la mesure d'un standard ou d'un échantillon par exemple), d'exporter les mesures enregistrées vers un logiciel de calcul de type Excel.

Le dispositif de l'invention communique avec le logiciel IHM par une liaison série RS-232. Le logiciel est exécutable en trois niveaux : un mode bas-niveau, un mode de contrôle à distance et un mode endurance autonome.
- En mode bas-niveau, chaque moyen et chaque paramètre est accessible manuellement. Par exemple, pour modifier la vitesse de rotation ou bien commuter ou non une vanne. Ce mode est utile pour les opérations de maintenance et de développements primaires.
- Dans le mode de contrôle à distance de l'analyseur, une programmation préenregistrée peut être lancée. Ce mode est utile par exemple pour les acquisitions d'une mesure sur le court terme ou lors d'un accès assez simple à l'analyseur.
- Dans le mode endurance autonome, une sélection de programmes, appelée également cycles, préenregistrés peut être lancée. Cette fonction est utile pour l'acquisition de longues séries de mesure ou pour des fonctionnements autonomes à heure fixe sur plusieurs jours par exemple. Les données peuvent être stockées dans une mémoire interne du dispositif de la présente invention et peuvent être extraites en utilisant le logiciel d'exploitation de l'analyseur (IHM). Le traitement des données brutes peut également être effectué par le logiciel pour obtenir la valeur de l'intensité lumineuse absorbée par les particules (turbidimétrie) et minimiser l'effet du bruit des mesures. Les données brutes et traitées peuvent ensuite être transférées à partir du logiciel IHM sur un logiciel de calcul EXCEL pour approfondir le traitement des données.

En outre, trois paramètres influant sur le signal optique sont réglables par un logiciel développé par les inventeurs : un paramètre « Conv » correspondant à la cadence de l'échantillonneur, autrement dit la fréquence de modulation des LEDs (« luminescent electronic device ») émettrices ; un paramètre « Range » correspondant à la variation du condensateur d'intégration du circuit imprimé permettant la détection synchrone ; et la « Puissance » des LEDs qui correspond à l'intensité lumineuse de chaque LED.

### Exemple 2 : Exemple de dispositif version côtier pour l'analyse d'une eau de mer nanofiltrée

Le dispositif de l'exemple 1 a été confiné dans une enceinte étanche comme représenté sur les figures 5a et 5b.

Sur la figure 5a, qui est une photographie de ce dispositif version côtier, on observe qu'il est composé d'un seul bloc, avec dans sa partie supérieure, les moyens de connexion du dispositif à la source d'eau de mer nanofiltrée pour son analyse.

Sur la figure 5b qui est une représentation schématique du dispositif côtier, on observe le connecteur (CAI) permettant l'apport du courant à l'appareil ainsi que la communication entre l'appareil et le logiciel.

Le module hydraulique, à savoir la boucle d'injection et de mélange selon l'invention, et le module optique/électronique, à savoir une cellule de détection (DC), 3 micro-LEDs, une photodiode et une carte électronique de détection ont été confinées au sein d'une enceinte étanche d'immersion sous-marine de hauteur 210 millimètres, de longueur 148 millimètres et de largeur 120 millimètres (figures 5a et 5b).

Cette enceinte est en PMMA (polyméthacrylate de méthyle) de 10 mm d'épaisseur. Le couvercle supérieur est de 25 mm d'épaisseur et le couvercle inférieur de 15 mm d'épaisseur. Elle a été obtenue par collage des quatre faces.

La figure 5b représente schématiquement le dispositif version côtier sans l'enceinte étanche.

Les moyens de connections entre le dispositif et la source d'eau de mer nanofiltrée est assurée au moyen d'un tuyau rigide.

L'étanchéité au niveau du passage de ces moyens de connexion par la partie supérieure de l'enceinte est assurée par des joints toriques en nitrile 70 shores A ou PB701 (marque commerciale, société Le Joint Français).

Ce dispositif a pu être immergé et a été fonctionnel jusqu'à une profondeur de 10 mètres.

### Exemple 3 : Dispositif version grands fonds pour l'analyse d'une eau de mer nanofiltrée

Le dispositif version grands fonds utilisé selon l'exemple 3 a été fabriqué conformément à la version côtière de l'exemple 2 en confinant d'une part le module hydraulique et le module optique/électronique dans deux enceintes distinctes et étanches (figure 7a).

L'étanchéité des deux modules a été assurée par la disposition du module hydraulique en équipression dans de l'huile diélectrique (Fluorinert (marque de commerce) (société 3M, USA), FC77, 3M, densité 1,78), tandis que le module optique/électronique a été disposé dans un caisson étanche en titane.

Un conduit lié au module hydraulique par un connecteur (COE) et lié au module optique/électronique par un connecteur (CMH) permet l'alimentation en énergie du module hydraulique et les actions de commande du module hydraulique (figure 8).

Le module optique/électronique dispose d'un connecteur (CAI) qui permet une communication à distance entre le logiciel et le dispositif et qui permet d'alimenter le dispositif en énergie.

L'enceinte du module optique/électronique comprend une carte électronique d'alimentation (CA) et une carte électronique de mesure (CEM) (figure 7e).

Ledit dispositif est immergeable jusqu'à 6000 mètres, et a été utilisé en opérationnel jusqu'à 2200 mètres.

Les dimensions du dispositif étant :
- pour le bloc hydraulique (MH) (figures 7b et 7c) : hauteur 130 mm, longueur 148 mm, largeur 120 mm,
- pour le bloc détection (MOE) (figure 7e) : hauteur 264 mm, diamètre 140 mm.

### Exemple 4 : Exemple d'utilisation du dispositif de la présente invention pour l'analyse in situ de sulfates dans une eau de mer nanofiltrée

Les expérimentations ont été réalisées avec le dispositif décrit dans l'exemple 2.

La source (S) du dispositif est connectée à un module de nanofiltration d'eau de mer via lesdites voies (V), l'eau de mer nanofiltrée constituant le liquide à analyser.

### Réactifs et solutions utilisées :

### Exemple de solution de mise en évidence des sulfates

La solution de mise en évidence des sulfates choisie est une solution de chlorure de Baryum (BaCl₂). Par agitation à l'aide d'un barreau aimanté, 0,20 g de cristaux de Thymol (5-méthyl-2-(propan-2-yl)-phénol) (Sigma-Aldrich, marque déposée, USA) ont été dissous dans 500 ml d'acide chlorhydrique (Sigma-Aldrich, marque déposée, USA) de concentration 0,005 mol.L⁻¹ à une température d'environ 80 °C. La solution a ensuite été refroidie à environ 40 °C et 1500 ml d'acide chlorhydrique de concentration 0,005 mol.L⁻¹ ont été ajoutés à la solution. 4 g de gélatine (Sigma-Aldrich, marque déposée, USA) ont été ajoutés doucement, puis le mélange a été agité jusqu'à la solubilisation de la gélatine. Enfin, 20 g de Chlorure de Baryum déshydraté (Sigma-Aldrich, marque déposée, USA) ont été dissous. Le mélange a été filtré et placé dans des poches de transfert.

### Exemple de solution de rinçage

La solution de rinçage préparée est une solution alcaline d'EDTA. Pour cela, 40 g d'EDTA (Fluka Analytical, marque déposée, USA), 7 g de chlorure d'ammonium (Sigma-Aldrich, marque déposée, USA) et 120 ml d'une solution concentrée d'ammoniac (Fluka Analytical, marque déposée, USA) ont été dissous dans 500 ml d'eau MilliQ (résistivité de 18,2 MΩ, température de 25 °C) (pH=10). Le mélange obtenu a ensuite été dilué jusqu'à 1 litre avec de l'eau déminéralisée.

### Exemple des solutions étalons de sulfates

Une solution mère de sulfates (5000 mg.L⁻¹) a été préparée en dissolvant 9,05 g de sulfate de potassium K₂SO₄ (Sigma-Aldrich, marque déposée, USA) dans un litre d'eau déminéralisée. Les solutions étalons (0, 10, 20, 30, 40, 50, 60, 70, 80, 90 et 100 mg.L⁻¹) ont été préparées en diluant un volume approprié de solution mère dans de l'eau déminéralisée ou dans une solution d'eau de mer synthétique sans sulfates ou solution d'eau de mer artificielle sans sulfates.

### Exemple de solution test : solution d'eau de mer synthétique sans sulfates

De l'eau de mer synthétique sans sulfates a été préparée en diluant dans 1900 ml d'eau déminéralisée les produits suivants :
Sels anhydres :
   - 49,06 g de NaCl
   - 0,2 g de KBr
   - 0,4 g de NaHCO₃
   - 1,4 g de KCl
   - 0,06 g de H₃BO₃
   - 0,06 g de NaF
Sels hydratés :
   - 22,2 MgCl₂, 6 H₂O
   - 3,08 g de CaCl₂, 2 H₂O
   - 0,34 g de SrCl₂, 6 H₂O

Le mélange obtenu a ensuite été complété à 2 litres avec de l'eau déminéralisée.

### Utilisation du dispositif de l'invention pour l'analyse de sulfates dans un liquide

Dans un premier temps, le liquide à analyser a été introduit dans le dispositif de la présente invention tel que décrit dans l'exemple 1, dans la boucle d'injection, puis a été entrainé par une pompe péristaltique A (PPA) pendant 80 secondes correspondante à la purge du circuit analytique.

Dans un second temps, la solution (R1) comprenant une quantité stœchiométrique de BaCl₂ a été introduite dans la boucle d'injection (BI) qui possède un volume prédéfini. Le réactif a ensuite été propulsé par la pompe péristaltique C (PPC). Enfin, l'échantillon a été remis en circulation par la pompe péristaltique A, mais a poussé cette fois-ci la solution comprenant une quantité stœchiométrique de BaCl₂ contenue dans la boucle d'injection.

Après réaction entre le liquide à analyser et le BaCl₂ dans la boucle de mélange, le précipité est passé devant le moyen d'analyse par turbidimétrie (CD) où il a été analysé avant d'être rejeté dans un contenant d'accueil du liquide analysé (non représenté). La formation du précipité a ensuite été détectée vers 420 nm pour la gamme 0 à 40 mg.L⁻¹ ou 810 nm pour la gamme 40 à 100 mg.L⁻¹.

En fin de cycle, un rinçage du circuit a été effectué en utilisant une solution alcaline d'éthylène diamine tétra acétate (EDTA) (R2) propulsée par la pompe péristaltique B.

Lorsque le liquide analysé contenait de faibles concentrations en sulfates (entre 0 et 60 mg.L⁻¹), le flux a été arrêté pendant 9 minutes lorsque ledit liquide à analyser et la solution comprenant du BaCl₂ étaient dans la boucle de mélange afin de détecter de plus faibles concentrations en sulfates et afin d'obtenir un signal plus sensible.

L'addition d'une solution alcaline d'EDTA a permis de dissoudre le précipité formé et a ainsi permis d'éviter le colmatage du circuit. L'ajout combiné de cristaux de thymol et de gélatine a permis de laisser en suspension les particules et donc d'éviter les dépôts sur les parois des tubes et dans la cellule de détection.

La gamme de mesure et la précision associée à cette gamme d'étude ont pu être modifiées en faisant varier la longueur d'onde des LEDs.

Tous les réactifs ont été préparés avec de l'eau Milli Q (Millipore (marque de commerce)).

Le dispositif d'analyse a été programmé et contrôlé par un logiciel d'exploitation Interface Homme Machine de l'analyseur développé sous Visual Basic et qui a pu être exécuté en trois niveaux : le mode bas-niveau, le mode de contrôle à distance et le mode endurance autonome.
- En mode bas-niveau, chaque moyen et chaque paramètre est accessible manuellement. Par exemple, pour modifier la vitesse de rotation ou bien commuter ou non une vanne. Ce mode est utile pour les opérations de maintenance et de développements primaires.
- Dans le mode de contrôle à distance de l'analyseur, une programmation préenregistrée peut être lancée. Ce mode est utile par exemple pour les acquisitions d'une mesure sur le court terme ou lors d'un accès assez simple à l'analyseur.
- Dans le mode endurance autonome, une sélection de programmes, appelée également cycles, préenregistrés peut être lancée. Cette fonction est utile pour l'acquisition de longues séries de mesure ou pour des fonctionnements autonomes à heure fixe sur plusieurs jours par exemple. Les données peuvent être stockées dans une mémoire interne du dispositif de la présente invention et peuvent être extraites en utilisant le logiciel d'exploitation de l'analyseur (IHM). Le traitement des données brutes peut également être effectué par le logiciel pour obtenir la valeur de l'intensité lumineuse absorbée par les particules (turbidimétrie) et minimiser l'effet du bruit des mesures. Les données brutes et traitées peuvent ensuite être transférées à partir du logiciel IHM sur un logiciel de calcul EXCEL pour approfondir le traitement des données.

### Mise au point des courbes d'étalonnage :

### • Courbes d'étalonnages pour des concentrations en sulfates comprises entre 0 et 100 mg/L dans les solutions étalon de sulfates

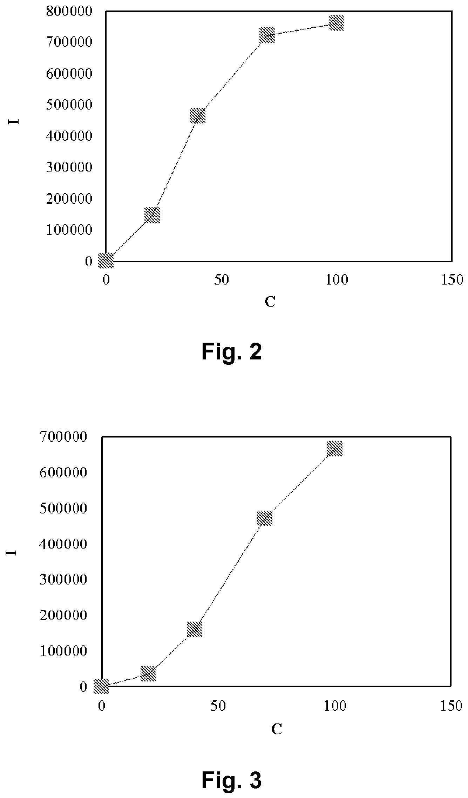

Des courbes d'étalonnage ont été établies selon le protocole expérimental décrit ci-dessus, à partir d'étalons de concentrations en sulfates de 0, 20, 40, 70 et 100 mg.L⁻¹ préparés comme ci-dessus, pour des longueurs d'ondes 420 nm (figure 2) et 810 nm (figure 3).

### • Courbes d'étalonnages pour des concentrations en sulfates comprises entre 0 et 60 mg/L

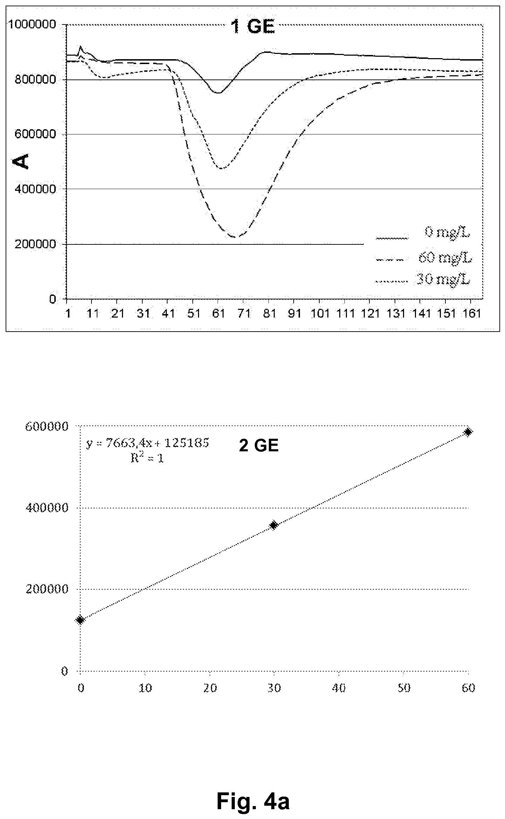

Des courbes d'étalonnages ont également été réalisées pour des valeurs de concentrations en sulfates comprises entre 0 et 60 mg.L⁻¹. L'analyse d'une gamme de standards de sulfates (0, 30 et 60 mg.L⁻¹) par le dispositif tel que décrit précédemment a donné les résultats qui sont représentés aux figures 4a et 4b. Ces courbes ont été obtenues avec l'utilisation d'un Stop Flow de 9 minutes après injection du réactif BaCl₂. Cet arrêt laisse plus de temps à la réaction pour se développer et permet ainsi une sensibilité accrue.

### Eau de mer nanofiltrée :

Une eau de mer de salinité 24,3 ‰ nanofiltrée a été obtenue après nanofiltration d'une eau de mer provenant de la mer Méditerranée par le pilote de la société Total installé sur la station Ifremer de Palavas-Les-Flots.

### Comparaison des résultats obtenus avec ceux obtenus par chromatographie ionique :

Une série de mesure a été réalisée en laboratoire pour l'obtention de concentrations sur quatre jours de manipulation sur une eau de mer nanofiltrée issue du pilote de nanofiltration de Palavas-Les-Flots. L'objectif étant de comparer les valeurs de concentrations obtenues par la méthode turbidimétrique par injection en flux continu inversé selon l'invention à celles obtenues par chromatographie ionique réalisées par le laboratoire Environnement Profond d'Ifremer Brest sur les quatre jours de manipulation.

### Comparaison des résultats obtenus sur plusieurs jours avec ceux obtenus par chromatographie ionique :

### • Valeurs obtenues par mesure par chromatographie ionique

Les échantillons ont été préparés par acidification à 1 %o en acide nitrique à 65 % puis dilution par 3 et par 5. Les échantillons ont été conservés à température ambiante.

Deux échantillons d'eau de mer nanofiltrée de salinité 24,3 ‰ ont été prélevés et analysés. La concentration moyenne après calcul avec le coefficient de dilution est de 90,1 mg.L⁻¹ avec un écart type de 1,5 mg.L⁻¹.

### • Valeurs obtenues par la méthode turbidimétrique par injection en flux continu inversé selon l'invention

Les mesures des quantités de sulfates contenues dans les liquides analysés ont été réalisées conformément au protocole expérimental décrit ci-dessus. Les résultats sont répertoriés dans le tableau 1 ci-dessous :

**Tableau 1: Concentrations moyennes pour chaque journée de la manipulation. Les écarts types sont signalés entre parenthèses.**

| Jour | Jour 1 | Jour 2 | Jour 3 | Jour 4 |
|---|---|---|---|---|
| Concentration moyenne (mg.L⁻¹) | 89,0 (5,5) | 89,9 (3,0) | 88,2 (3,0) | 90,1 (3,5) |

Dans un premier temps, les résultats obtenus montrent que les valeurs des concentrations mesurées de l'échantillon sont proches puisqu'elles varient entre 88,2 et 90,1 mg.L⁻¹. De plus, ces valeurs sont également proches de la valeur déterminée par chromatographie ionique qui était de 90,1 mg.L⁻¹.

Par conséquent, la méthode possède des performances analytiques en accord avec les spécifications demandées.

### Performances analytiques de l'analyseur

Les performances analytiques de l'analyseur ont été évaluées selon le protocole expérimental décrit ci-dessus. Les résultats sont répertoriés dans le tableau 2 ci-dessous :

**Tableau 2: Performances analytiques de l'analyseur selon l'invention.**

| Gamme | Limite de détection | Limite de quantification | Précision (à 10 mg.L⁻¹) (à 40 mg.L⁻¹) |
|---|---|---|---|
| 0 - 60 mg.L⁻¹ | 4 mg.L⁻¹ | 10 mg.L⁻¹ | 5% - 1,1 % |

Les résultats montrent que la limite de quantification et la limite de détection sont en accord avec le critère de concentrations en sulfate tolérées dans l'utilisation de l'eau de mer nanofiltrée dans l'exploitation des gisements, la valeur seuil étant autour de 40 mg.L⁻¹.

La précision (ou « coefficient de variabilité » défini comme le rapport entre l'écart-type et la moyenne multiplié par 100) obtenue pour un standard de 10 mg.L⁻¹ est de 5 % (acceptable pour un analyseur *in situ*) et elle est de 1,1% pour un standard à 40 mg.L⁻¹ (valeur cible). Ces paramètres ont été obtenus en travaillant dans la gamme 0 à 60 mg.L⁻¹.

### Liste des références

**[1]** Van Staden, 1987, On-line sulfate monitoring by reversed flow injection analysis and alternating reagent injection, Fresenius Z Anal Chem 326: 754-756.
**[2]** Vuillemin et al, 2009, CHEMINI : A new in situ CHEmical MINIaturized analyzer, Elsevier, Deep-Sea Research I 56(2009) 1391-1399.

## Revendications

1. Dispositif (1) d'analyse de sulfates dans un liquide (L), ledit dispositif comprenant les éléments suivants :
**a)** un circuit analytique (3) étanche comprenant une boucle d'injection (BI) de volume défini connectée à deux, trois ou quatre boucles de mélange (BM), de préférence deux boucles de mélange, qui se suivent, lesdites boucles de mélange étant ondulées ou enroulées en spirale, de préférence enroulées en spirale , et un moyen d'analyse par turbidimétrie (CD), la boucle d'injection, les boucles de mélange et le moyen d'analyse pouvant être parcourus successivement en continu par le liquide, la boucle d'injection et la boucle de mélange étant séparées par une vanne (VH) ;
**b)** un moyen d'injection (IL) dudit liquide dans ladite boucle d'injection, ledit moyen d'injection comprenant une première pompe péristaltique (PPA), un système de vannes (VF, VG) placées entre ladite première pompe (PPA) et ladite boucle d'injection, et un moyen de prélèvement dudit liquide à partir d'une source (S) ;
**c)** un moyen d'injection (ISR), dans ladite boucle d'injection, d'une solution de mise en évidence de sulfates dans ledit liquide par turbidimétrie ; ledit moyen d'injection (ISR) comprenant une deuxième pompe péristaltique (PPC) d'injection de ladite solution de mise en évidence de sulfates et une vanne (VG) placée entre ladite deuxième pompe (PPC) et ladite boucle d'injection ; et
**d)** un moyen d'injection (ISP) d'une solution de rinçage dudit circuit analytique, ledit moyen d'injection (ISP) comprenant une troisième pompe péristaltique (PPB) d'injection de ladite solution de rinçage et un système de vannes (VF, VG) placées entre ladite troisième pompe (PPB) et ladite boucle d'injection.

2. Dispositif selon la revendication 1, dans lequel le moyen de prélèvement dudit liquide à partir de la source (S) comprend au moins une voie (V) de prélèvement du liquide en mouvement, ladite voie comprenant au moins deux vannes (VA, VB, VC, VD, VE) placées entre ledit liquide en mouvement et la première pompe péristaltique (PPA).

3. Dispositif selon la revendication 2, dans lequel ladite source est connectée à un module de nanofiltration d'eau de mer via ladite voie (V), l'eau de mer nanofiltrée constituant ledit liquide (L) en mouvement.

4. Dispositif selon la revendication 1, ledit dispositif étant automatisé par des moyens de programmation et de contrôle d'injection en flux continu inversé, dans ladite boucle d'injection, de la solution de mise en évidence de sulfates dans ledit liquide, ladite solution comprenant une quantité stœchiométrique de BaCl₂ permettant de détecter une concentration maximale de sulfates acceptable dans ledit liquide.

5. Dispositif selon la revendication 1 comprenant en outre une mémoire permettant de mémoriser des analyses successives.

6. Dispositif selon la revendication 1 comprenant en outre un logiciel d'exploitation Interface Homme-Machine développé sous Visual Basic.

7. Dispositif selon l'une quelconque des revendications précédentes, ledit dispositif étant composé de deux parties, l'une comprenant les boucles d'injection et de mélange et l'autre comprenant le moyen d'analyse par turbidimétrie.

8. Appareil comprenant au moins une enceinte étanche d'immersion sous-marine et un dispositif selon l'une quelconque des revendications précédentes, ledit dispositif étant confiné au sein de ladite au moins une enceinte étanche d'immersion sous-marine.

9. Utilisation d'un dispositif tel que défini dans l'une quelconque des revendications 1-7 pour l'analyse de sulfates dans une eau de mer nanofiltrée, ledit liquide (L) étant ladite eau de mer nanofiltrée.

10. Utilisation selon la revendication 9, dans laquelle l'eau de mer nanofiltrée est celle injectée dans une cavité pour extraire du pétrole, la voie de prélèvement (V) permettant d'échantillonner *in situ* cette eau de mer nanofiltrée pour en analyser son contenu en sulfates.

11. Utilisation selon la revendication 9 ou 10, ladite utilisation comprenant les étapes suivantes :
a) injection en flux continu inversé, dans ladite boucle d'injection, de solution de mise en évidence de sulfates dans ledit liquide, ladite solution comprenant une quantité stœchiométrique de BaCl₂ permettant de détecter une concentration maximale de sulfates acceptable dans ledit liquide par formation de BaSO₄, ladite solution réactive pouvant comprendre en outre des cristaux de thymol et de la gélatine ;
b) injection dudit liquide (L) à analyser dans ladite boucle d'injection, ledit liquide à analyser poussant ladite solution injectée à l'étape a) et se mélangeant à celle-ci dans la boucle de mélange ; et
c) mesure par turbidimétrie du BaSO₄ formé le cas échéant lors de l'étape b) via le moyen d'analyse par turbidimétrie (CD).

12. Utilisation selon la revendication 11, dans laquelle l'étape a) est précédée d'une étape de purge du circuit au moyen du liquide (L) en le faisant circuler grâce à la première pompe péristaltique (PPA) dans les boucles d'injection et de mélange et dans le moyen d'analyse par turbidimétrie.

13. Utilisation selon la revendication 11 ou 12, dans laquelle l'étape c) de mesure est suivie d'une étape de rinçage du circuit analytique par injection dans celui-ci d'une solution de rinçage éliminant le BaSO₄ formé le cas échéant, ladite solution de rinçage pouvant comprendre de l'EDTA.

14. Utilisation selon l'une quelconque des revendications 11 à 13, dans laquelle on procède à un étalonnage du dispositif au moyen d'un ou de plusieurs standard(s) préparé(s) avec de l'eau de mer artificielle.

15. Utilisation d'un dispositif selon l'une quelconque des revendications 1 à 7 pour l'analyse de sulfate dans un liquide destiné à ou issu d'un procédé industriel.

## Patentansprüche

1. Vorrichtung (1) zur Analyse von Sulfaten in einer Flüssigkeit (L), wobei die Vorrichtung die folgenden Elemente umfasst:
**a)** einen dichten Analysekreislauf (3), der eine Einspritzschleife (BI) mit definiertem Volumen, die mit zwei, drei oder vier aufeinander folgenden Mischschleifen (BM), vorzugsweise zwei Mischschleifen, verbunden ist, wobei die Mischschleifen gewellt oder spiralförmig gewickelt, vorzugsweise spiralförmig gewickelt, sind, und ein turbidimetrisches Analysemittel (CD) umfasst, wobei die Einspritzschleife, die Mischschleifen und das Analysemittel nacheinander und kontinuierlich von der Flüssigkeit durchströmt werden können, wobei die die Einspritzschleife und die Mischschleife durch ein Ventil (VH) getrennt sind;
**b)** ein Mittel zur Injektion (IL) der Flüssigkeit in die Einspritzschleife, wobei das Mittel zur Injektion eine erste peristaltische Pumpe (PPA), ein System von Ventilen (VF, VG), die zwischen der ersten Pumpe (PPA) und der Einspritzschleife angeordnet sind, und ein Mittel zur Entnahme von Flüssigkeit aus der Quelle (S);
**c)** ein Mittel zum Einspritzen (ISR) einer Lösung zum Nachweis von Sulfaten in der Flüssigkeit durch Turbidimetrie in die Einspritzschleife; wobei die Mittel zum Einspritzen (ISR) eine zweite peristaltische Pumpe (PPC) zum Einspritzen der Lösung zum Nachweis von Sulfaten und ein Ventil (VG), das zwischen der zweiten Pumpe (PPC) und der Einspritzschleife angeordnet ist, umfassen; und
**d)** ein Mittel zum Einspritzen (ISP) einer Spüllösung für den analytischen Kreislauf, wobei die Einspritzmittel (ISP) eine dritte peristaltische Pumpe (PPB) zum Einspritzen der Spüllösung und ein System von Ventilen (VF, VG) umfassen, die zwischen der dritten Pumpe (PPB) und der Einspritzschleife angeordnet sind.

2. Vorrichtung nach Anspruch 1, wobei das Mittel zur Entnahme der Flüssigkeit aus der Quelle (S) mindestens einen Weg (V) zum Abziehen der sich bewegenden Flüssigkeit umfasst, wobei der Weg mindestens zwei Ventile (VA, VB, VC, VD, VE) umfasst, die zwischen der sich bewegenden Flüssigkeit und der ersten peristaltischen Pumpe (PPA).

3. Vorrichtung nach Anspruch 2, wobei die Quelle über den Weg (V) mit einem Meerwasser-Nanofiltrationsmodul verbunden ist, wobei das nanofiltrierte Meerwasser die bewegte Flüssigkeit (L) bildet.

4. Vorrichtung nach Anspruch 1, wobei die Vorrichtung durch Mittel zum Programmieren und Steuern der Injektion in kontinuierlichem Gegenstrom in die Injektionsschleife der Lösung zum Nachweis von Sulfaten in der Flüssigkeit automatisiert ist, wobei die Lösung eine stöchiometrische Menge BaCI2 umfasst, die den Nachweis einer maximal akzeptablen Konzentration von Sulfaten in der Flüssigkeit ermöglicht.

5. Vorrichtung nach Anspruch 1, die ferner einen Speicher zum Speichern aufeinanderfolgender Analysen.

6. Vorrichtung nach Anspruch 1, die ferner eine unter Visual Basic entwickelte Bediensoftware für die Mensch-Maschine-Schnittstelle umfasst.

7. Vorrichtung nach einem der vorhergehenden Ansprüche, wobei die Vorrichtung aus zwei Teilen besteht, von denen einer die Einspritz-und Mischschleifen und der andere die turbidimetrischen Analysemittel umfasst.

8. Vorrichtung, die mindestens ein abgedichtetes Unterwasser-Tauchgehäuse und eine Vorrichtung nach einem der vorhergehenden Ansprüche umfasst, wobei die Vorrichtung innerhalb des mindestens einen abgedichteten Unterwasser-Tauchgehäuses eingeschlossen ist.

9. Verwendung einer Vorrichtung nach einem der Ansprüche 1-7 zur Analyse von Sulfaten in nanofiltriertem Meerwasser, wobei die Flüssigkeit (L) das nanofiltrierte Meerwasser ist.

10. Verwendung nach Anspruch 9, wobei das nanofiltrierte Meerwasser dasjenige ist, das in einen Hohlraum zur Ölextraktion injiziert wird, wobei der Probenahmeweg (V) eine In-situ-Probenahme dieses nanofiltrierten Meerwassers ermöglicht, um seinen Sulfatgehalt zu analysieren.

11. Verwendung nach Anspruch 9 oder 10, wobei die Verwendung die folgenden Schritte umfasst:
a) Injizieren einer Sulfatnachweislösung in die Flüssigkeit in kontinuierlichem Rückfluss in die Einspritzschleife, wobei die Lösung eine stöchiometrische Menge BaCl₂ umfasst, die es erlaubt, eine maximale akzeptable Konzentration von Sulfaten in der Flüssigkeit durch Bildung von BaSO₄ nachzuweisen, wobei die Reagenzlösung weiterhin Thymolkristalle und Gelatine umfassen kann;
b) Einspritzen der zu analysierenden Flüssigkeit (L) in die Einspritzschleife, wobei die zu analysierende Flüssigkeit die in Schritt a) eingespritzte Lösung schiebt und sich mit ihr in der Mischschleife vermischt; und
c) turbidimetrisches Messen des in Schritt b) gebildeten BaSO₄ über die turbidimetrische Analyseeinrichtung (CD).

12. Verwendung nach Anspruch 11, bei der dem Schritt a) ein Schritt des Spülens des Kreislaufs mittels der Flüssigkeit (L) durch Umwälzung durch die erste peristaltische Pumpe (PPA) in den Einstpriz- und Mischschleifen und in der Trübungsanalyseeinrichtung vorausgeht.

13. Verwendung nach Anspruch 11 oder 12, wobei auf Schritt c) des Messens ein Schritt des Spülens des Analysekreislaufs folgt, indem eine Spüllösung hineingespritzt wird, die das gebildete BaSO₄ entfernt, falls vorhanden, wobei die Spüllösung EDTA umfassen kann.

14. Verwendung nach einem der Ansprüche 11 bis 13, wobei eine Kalibrierung der Vorrichtung mittels eines oder mehrerer mit künstlichem Meerwasser hergestellter Standards durchgeführt wird.

15. Verwendung einer Vorrichtung nach einem der Ansprüche 1 bis 7 zur Analyse von Sulfat in einer Flüssigkeit, die für einen industriellen Prozess bestimmt ist oder daraus resultiert.

## Claims

1. Device (1) for the analysis of sulfates in a liquid (L), said device comprising the following elements:
**a)** a sealed analytical circuit (3) comprising an injection loop (BI) of defined volume connected to two, three or four mixing loops (BM), preferably two mixing loops, which follow one another, said mixing loops being corrugated or spirally wound, preferably spirally wound, and a turbidimetric analysis means (CD), the injection loop, the mixing loops and the analysis means being able to be successively and continuously flowed through by the liquid, the injection loop and the mixing loop being separated by a valve (VH);
**b)** means for injecting (IL) said liquid into said injection loop, said injection means comprising a first peristaltic pump (PPA), a system of valves (VF, VG) placed between said first pump (PPA) and said injection loop, and means for taking said liquid from a source (S)
**c)** means for injecting (ISR), into said injection loop, a solution for detecting sulfates in said liquid by turbidimetry; said injection means (ISR) comprising a second peristaltic pump (PPC) for injecting said solution for detecting sulfates and a valve (VG) placed between said second pump (PPC) and said injection loop; and
**d)** means for injecting (ISP) a rinse solution for said analytical circuit, said injecting means (ISP) comprising a third peristaltic pump (PPB) for injecting said rinse solution and a system of valves (VF, VG) located between said third pump (PPB) and said injection loop.

2. The device according to claim 1, wherein the means for withdrawing said liquid from the source (S) comprises at least one path (V) for withdrawing the moving liquid, said path comprising at least two valves (VA, VB, VC, VD, VE) placed between said moving liquid and the first peristaltic pump (PPA).

3. The device of claim 2, wherein said source is connected to a seawater nanofiltration module via said pathway (V), the nanofiltered seawater constituting said moving liquid (L).

4. A device according to claim 1, said device being automated by means for programming and controlling the injection in continuous reverse flow, into said injection loop, of the solution for detecting sulfates in said liquid, said solution comprising a stoichiometric amount of BaCl₂ allowing the detection of a maximum acceptable concentration of sulfates in said liquid.

5. The device of claim 1 further comprising a memory for storing successive analyses.

6. A device according to claim 1 further comprising a Human Machine Interface operating software developed under Visual Basic.

7. Device according to any one of the preceding claims, said device being composed of two parts, one comprising the injection and mixing loops and the other comprising the turbidimetric analysis means.

8. An apparatus comprising at least one sealed underwater immersion enclosure and a device according to any of the preceding claims, said device being confined within said at least one sealed underwater immersion enclosure.

9. Use of a device as defined in any one of claims 1-7 for the analysis of sulfates in nanofiltered seawater, said liquid (L) being said nanofiltered seawater.

10. The use according to claim 9, wherein the nanofiltered seawater is that injected into a cavity to extract oil, the sampling pathway (V) allowing in situ sampling of this nanofiltered seawater to analyze its sulfate content.

11. The use of claim 9 or 10, said use comprising the following steps:
a) injecting in continuous reverse flow, into said injection loop, sulfate detection solution in said liquid, said solution comprising a stoichiometric amount of BaCl₂ allowing to detect a maximum acceptable concentration of sulfates in said liquid by formation of BaSO₄, said reagent solution may further comprise thymol crystals and gelatin;
b) injecting said liquid (L) to be analyzed into said injection loop, said liquid to be analyzed pushing said solution injected in step a) and mixing with it in the mixing loop; and
c) turbidimetrically measuring the BaSO₄ formed in step b) via the turbidimetric analysis means (CD).

12. Use according to claim 11, in which step a) is preceded by a step of purging the circuit by means of the liquid (L) by circulating it through the first peristaltic pump (PPA) in the injection and mixing loops and in the turbidimetry analysis means.

13. Use according to claim 11 or 12, wherein step c) of measuring is followed by a step of rinsing the analytical circuit by injecting therein a rinsing solution removing the BaSO₄ formed if any, said rinsing solution may comprise EDTA.

14. The use according to any one of claims 11 to 13, wherein a calibration of the device is carried out by means of one or more standards prepared with artificial seawater.

15. Use of a device according to any one of claims 1 to 7 for the analysis of sulfate in a liquid intended for or resulting from an industrial process.
